(19) Europäisches Patentamt

European Patent Office

Office européen des brevets

(11) **EP 0 535 327 B1**

(12) **EUROPEAN PATENT SPECIFICATION**

(45) Date of publication and mention
of the grant of the patent:
**23.10.1996 Bulletin 1996/43**

(51) Int. Cl.⁶: **A61K 9/12**, A61K 47/00,
A61K 47/18

(21) Application number: **92113209.8**

(22) Date of filing: **03.08.1992**

(54) **Pharmaceutical composition containing felbinac**

Felbinac enthaltendes Arzneimittel

Composition à base de Felbinac

(84) Designated Contracting States:
**AT BE CH DE DK ES FR GR IE IT LI LU NL PT SE**

(30) Priority: **01.10.1991 GB 9120831**

(43) Date of publication of application:
**07.04.1993 Bulletin 1993/14**

(73) Proprietor: **AMERICAN CYANAMID COMPANY**
**Stamford Connecticut 06904-0060 (US)**

(72) Inventors:
• **Ramsey, Michael Peter**
**Fareham, Hampshire, PO16 8DN (GB)**

• **Saunders, Richard William**
**Gosport, Hampshire, PO12 2EP (GB)**

(74) Representative: **Allam, Peter Clerk et al**
**LLOYD WISE, TREGEAR & CO.,**
**Commonwealth House,**
**1-19 New Oxford Street**
**London WC1A 1LW (GB)**

(56) References cited:
**EP-A- 0 171 084          EP-A- 0 214 501**
**EP-A- 0 313 347**

• **STN Information Services Data Base: Chemical**
**Abstract Accession Number: 115 (16) ,166660 h**

## Description

This invention relates to a pharmaceutical composition containing 4-biphenylacetic acid (BPAA), also sometimes known as felbinac, for topical delivery in the treatment of joint trauma (e.g. sprains, strains and other soft tissue injuries) and other conditions where a reduction in inflammation is required.

BPAA is the active metabolite of fenbufen, a drug of the non-steroidal anti-inflammatory class (i.e. an NSAID). BPAA itself, however, has hitherto not generally been used in therapy, although recently there has become available a BPAA-containing gel for topical application in the treatment of arthritis. BPAA gels are, however, subject to certain limitations.

In particular, it would be desirable to be able to enhance the absorbability of the active ingredient in the period immediately following application, in order that the patient should obtain more rapid relief from the, sometimes quite acute, pain which often accompanies soft tissue injuries, for example.

JP-A-03072433, which is abstracted in STN Information Services Data Base: Chemical Abstract Accession Number 115(16), 166660h, discloses foaming aerosol preparations of non-steroidal anti-inflammatory and analgesic agents, including BPAA, which are useful for the topical treatment of various painful conditions. The preparations are formulated so as to produce fine foams which cause low irritation on nasal mucosa.

In accordance with the present invention it has now been unexpectedly discovered that a quick break foam formulation of BPAA provides a dramatic enhancement in absorbability in the first few hours following administration as compared with a BPAA gel.

Thus, in accordance with the present invention there is provided a pharmaceutical composition suitable for topical application and comprising BPAA or a pharmaceutically acceptable salt or ester thereof, in an amount of from 0.1 to 20% w/w calculated as the free acid, and (b) a carrier which comprises water, ethanol, an aerosol propellent and an ethoxylated stearyl alcohol surfactant, the relative proportions of said components of said carrier being such that the carrier is adapted to form a quick-breaking foam; the composition being maintained under pressure.

The active ingredient in the present composition may be 4-biphenylacetic acid itself, or it may be a pharmaceutically acceptable salt or ester thereof. Examples of suitable inorganic salts are the sodium and calcium salts, while the ethanolamine salt is an example of a useful organic salt, and examples of useful, pharmaceutically acceptable esters include the ethyl ester. For convenience, the abbreviation "BPAA" will be used hereafter to refer to the active ingredient in any of its possible forms unless the context otherwise requires.

As is known to those skilled in the art of formulating aerosol foams, quick-breaking foam systems are designed to foam when discharged by propellant from the container in which they are maintained under pressure but the foam breaks down rapidly when heated by skin contact and/or is subject to shear forces, as by being rubbed in. Such foam systems have been quite widely used in the formulation of cosmetic preparations, but hitherto they have not found widespread use as delivery vehicles for therapeutic agents, notwithstanding the paper by Woodford & Barry in J. Pharm. Sci. 66 No. 1 (January 1977) and in which these authors suggest that quick break foams are suitable vehicles for topical corticosteroids and may be a useful delivery system for other medicaments as well. Actually, inspection of the experimental data presented by Woodford & Barry shows that the absorbability of the corticosteroids which were tested was only slightly improved when the active ingredient was formulated as a quick break foam, as compared with such conventional semi-solid formulations as ointments and gels (there was a greater improvement when foam concentrates were tested but such are not of any practical value).

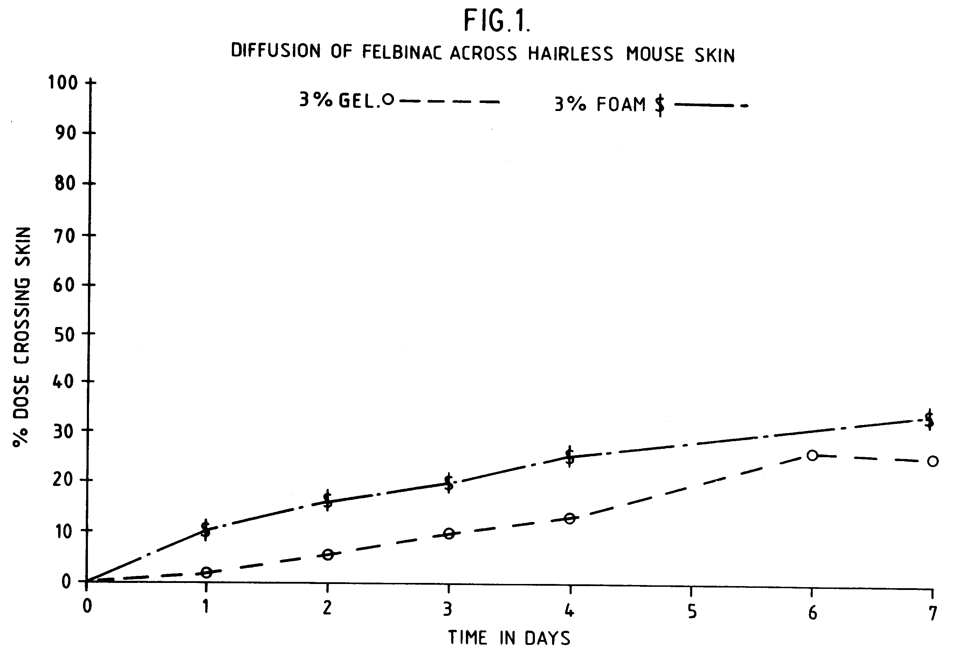

More particularly, in none of the experiments reported by Woodford & Barry was there any significant increase in absorbability in the period immediately following administration of the test composition. For instance, the skin blanching curves given in Fig. 1 on page 100 of the paper show substantially identical profiles for all the different types of formulation which measured by the skin blanching response were tested, with maximum absorbability as being evident about 15 hours post-administration.

In contrast, preferred BPAA-containing quick break foams in accordance with the present invention show a maximum increase in absorbability, as compared with an equivalent BPAA-gel, within the first two or three hours following administration, as especially evidenced by the in vivo tests in rabbits described in Example 5 below.

Although it is believed that those skilled in the art of aerosol formulations can readily distinguish a quick-breaking foam from a more conventional foam, for the avoidance of doubt a foam is considered by us to be "quick-breaking" if a 2 g sample of the foam breaks down completely within 5 minutes (preferably within 3 minutes) on a horizontal tile maintained at 37°C.

Thus, the present invention is predicated on our discovery that BPAA is absorbed by the skin from a quick-breaking foam, in a surprisingly rapid manner, whereby it is possible to provide a product which can be readily applied as an aerosol foam from a suitable pressurized container for the rapid relief of painful inflammation.

The carrier used in the present pharmaceutical composition must be one which possesses quick-break foaming properties when the composition is discharged from the container in which it is stored under pressure. In general, the quick-break aerosol foam systems currently available comprise a water-ethanol base with a high alcohol concentration, an aerosol propellant and an ethoxylated stearyl alcohol surfactant which, when the foam is dispensed, initially precipitates out of the aqueous alcohol to stabilize the foam, as a result of the evaporation of the propellant, but then dissolves again in the solvent base on the foam being subjected to heating and/or shear forces. In order to form such a quick-break composition it is necessary that the relative proportions of the four components, viz water, ethanol, ethoxylated stearyl alcohol surfactant and propellant, should be controlled within relatively close limits. A detailed discussion of the properties of aqueous alcohol aerosol foam system is given in a two-part article by Paul A. Sanders in Drug and Cosmetic Industry, 99 (2), pages 56-154 (1966) and 99 (3), pages 56-154 (1966).

Particularly suitable surfactants for use in quick break foams of the present invention are the ethoxylated stearyl alcohols available under the trade names Polawax A31 and Brij 72.

A hydrocarbon aerosol propellant is preferred for environmental reasons, for example a mixture of propane, n-butane and iso-butane has been found to be suitable.

The concentration of the BPAA in the present pharmaceutical compositions is not critical and is limited only by its solubility in the carrier, but in general the BPAA is present in an amount of from 0.1% to 20% w/w. It is, indeed, an advantage of the present invention that it can provide a topical BPAA formulation with relatively high, i.e. above about 5% w/w of the active ingredient, thereby potentially opening the way for other uses, e.g. in the treatment of osteoarthritis. In contrast, with current gel formulations the BPAA concentration cannot exceed about 5% w/w.

In order to ensure that the BPAA is completely solubilized in the carrier, it is preferred to incorporate the active ingredient either as a pre-formed salt or alternatively and more preferably to add a suitable base to the formulation in order to form a soluble BPAA salt in situ. Suitable bases include ethanolamine, diethanolamine, diisopropanolamine, triethanolamine, TRIS (i.e. 2-amino-2-(hydroxymethyl)-propane-1,3-diol) and certain amino acids, ethanolamine being currently the base of choice.

If desired, other ingredients such as emollient agents to provide an improved skin feel, agents to promote skin hydration, absorption enhancers and fragrances may be incorporated into the compositions of this invention.

The preferred procedure for formulating the compositions of this invention involves first preparing an alcoholic concentrate by dissolving the surfactant, BPAA, base such as ethanolamine and any optional ingredients in ethyl alcohol. The thus obtained concentrate and a pre-weighed amount of purified water are now separately charged into open aerosol containers, whereafter the actuator-valve assemblies are applied to the containers to close them. Finally, the selected propellant is added through the actuator valve.

It is not normally necessary to take special steps to sterilize the compositions of this invention, since they generally will not be applied to broken skin.

Any suitable aerosol container may be used for the compositions of the present invention. However, as BPAA is an acid, it is preferred that if a metal can is used it should be internally coated, as with a lacquer, in order to prevent corrosion.

The compositions of the present invention have a number of advantages, in addition to the high initial rate of absorption of the BPAA and the ability to incorporate large concentrations of the active ingredient to which reference has already been made. Thus, the compositions can be easily and directly applied to the affected part of the patient, and the applied foam will not run when first dispensed, unlike a liquid formulation. Dosage can be approximately controlled by the size of foam aliquot applied, or the containers can be provided with actuators which disperse a more precise, predetermined, amount of the foam. Finally, as the compositions are contained under pressure, there is no risk of the ingress of contaminants.

The invention is illustrated by the Examples which follow.

Example 1

A composition containing 3.17% w/w of BPAA active in a quick-break foam carrier was prepared as follows:
To a suitable clean and dry vessel was added 33.3 g Polawax A31 (surfactant) and 534 g ethanol. The contents were stirred at 15°-25°C until the Polawax had completely dissolved and a clear solution had formed. Whilst maintaining the temperature at 15°-25°C, 9.6 g ethanolamine BP and 50.0 g Softigen 767 (emollient) were added to the solution and stirring was continued until these ingredients had also dissolved. The preparation of the alcoholic concentrate was completed by mixing in 33.3 g of BPAA until the solution was clear again.

Into a separate vessel was weighed out 333.6 g of purified water BP. The alcoholic concentrate and the water were now charged into 80 g open, internally lacquered tinplate aerosol cans, and then the actuator-valve assemblies were crimped into position to close the cans. Finally, the cans were charged with 5% w/w butane 40 propellant through the actuator-valve assemblies. Butane 40 consists of 54% butane, 22% propane and 24% isobutane, all by weight.

The final composition of the pressurized formulation was as follows:

| Ingredient | % w/w |
|---|---|
| BPAA | 3.3[1] |
| Ethanolamine | 0.96 |
| Ethanol | 53.4 |
| Water | 33.3 |
| Polawax A31[2] | 3.97 |
| Softigen 767[3] | 5.0 |

[1] allowing for the 5% w/w of propellant, each gram of discharged foam will contain about 3.2% w/w of BPAA.
[2] Polawax A31 is an ethoxylated stearyl alcohol surfactant
[3] Softigen 767 is an emollient and consists of a water-soluble mixture of partial glycerides of natural $C_{8-12}$ saturated vegetable fatty acids which incorporates ethylene oxide

Upon discharge from the pressurized aerosol cans a foam was produced which broke down within about 60 seconds following application to the skin surface.

Example 2

A pressurized composition was prepared in a similar way to that described in Example 1 except that as the propellant there was used 10% w/w of a chlorofluorocarbon (CFC 12:114, (40:60)).

The foam produced from this composition broke down within about 60 seconds at 37°C.

The CFC-containing composition of this example, although entirely satisfactory from the point of view of their therapeutic properties, is nonetheless less preferred than the composition of Example 1 because of the environmental damage associated with CFCs.

Example 3

An in vitro study was carried out to assess the rate of transport of BPAA across excised hairless mouse skin in a diffusion cell from (a) a ~ 3% w/w BPAA foam formulation of this invention and (b) a ~ 3% w/w BPAA gel formulation.

Each skin patch was fitted into a holder so that a constant surface area was available in every case for diffusion. The skin and its holder were tightly clamped together with a glass diffusion cell. The latter was filled with 10 ml of a 0.9% w/v sodium chloride solution containing 0.002% w/v mercuric chloride as a preservative. Care was taken that the solution was fully in contact with the skin section and that no air bubbles were present.

A known dose of the BPAA preparation to be studied was placed into the well of the skin holder so that it was in contact with the upper skin surface. In order to apply a sufficient dose of the foams it was necessary to firstly break the foam before pipetting a sample into the well.

The well was sealed off from the atmosphere by placing a greased microscope slide over it. The sampling arm of the diffusion cell was sealed with a strip of parafilm.

The experiment was carried out in an oven maintained at 32°C.

Each cell was sampled at 24 hourly intervals. 1 ml of the receptor solution was withdrawn via the side arm and filtered through a 0.45 µ filter. The sample removed was replaced with a fresh 1 ml of receptor phase.

Sample assay was performed by HPLC.

Mobile phase; (filtered through a 0.45 µ filter)

Buffer 55%
Methanol 45%

Buffer composition

| Potassium dihydrogen orthophosphate | 7.48 g |
|---|---|
| Sodium hydroxide | 1.54 g |
| Distilled water          to | 2000 ml |

Flow rate; 1 ml/min
Column; "Partisil" - 10 ODS*

12 cm long x 4.6 mm I.D.

Detection wavelength 254 nm

Sample dilution; 0.4 ml to 10 ml with mobile phase (The 24 hour gel samples were assayed undiluted)

The composition of the test foam and gel preparations is given in Table I. The test foam was prepared by a similar procedure to that of Example 1.

The amount of BPAA that had diffused through the skin was calculated as a percentage of the dose initially applied. The results are shown graphically in Fig. 1.

*consists of silica spheres of 10 micron diameter and with octadecylsilane moieties attached

Table I

| | Formulation Type | Ingredients | % w/w |
|---|---|---|---|
| 1. | BPAA Foam | BPAA | 3.333 |
| | | Ethanolamine | 0.959 |
| | | Ethanol BP 96% | 53.391 |
| | | Water | 33.357 |
| | | Polawax 31 | 3.960 |
| | | Softigen 767 | 5.000 |
| | | Butane 40 | 5.000 |
| 2. | BPAA Gel | BPAA | 3.00 |
| | | Carbopol 940[1] | 1.00 |
| | | Diisopropanolamine (90% Solution) | 3.78 |
| | | Ethanol BP 96% | 30.79 |
| | | Purified Water | 61.43 |

[1] carboxypolymethylene

From that graph of Fig. 1 it will be noted that there was a significantly higher initial rate of diffusion from the BPAA foam than from the BPAA gel. Thus, for example, it will be noted that after one day about 10% of the dose had crossed the mouse skin from the foam, whereas that level was not reached until after about 4 days with the gel formulation.

Example 4

In this experiment the permeation characteristics of BPAA for a 3% foam formulation were compared with those of a 3% gel formulation. The foam and gel formulations had the same compositions as those used in Example 3. The gel was applied as formulated, whereas the foam was expelled into a glass vessel, stoppered and warmed to 37°C to break the foam before applying.

Suitable specimens of human abdominal skin, obtained at post-mortem and stored frozen at -24°C, were selected. Full thickness membranes were prepared by clamping flat the slightly thawed skin between two metal plates which were then placed in a freezer at -24°C for two hours to refreeze the skin. The top plate was removed and the epidermal surface of the skin allowed to thaw slightly until it was just mobile to the touch. A layer 430 $\mu$m$\pm$ 5% was cut using a Davies Dermatone 7 (Duplex Electro Dermatome). This dermatomed layer consisted of the epidermis and some of the dermal tissue.

Membranes prepared as outlined above (F-71yr,M-64,F-52,M-53,F-87) were subdivided to provide matched specimens for both the foam and gel diffusion experiments. These were allowed to hydrate overnight, by floating on 0.001% mercuric chloride solution in normal saline, before being mounted in 16 mm diameter glass diffusion cells providing a diffusional area of 2 cm$^2$. Receptor solution was placed in the donor and receptor compartments of the cells which were covered and subsequently placed in a water bath so that the receptor was at 37°C and the donor was exposed to 22°C (temperature controlled room). Three days were sufficient to allow the membranes to hydrate fully.

At the conclusion of the hydration period, receptor solution was removed from the donor compartment and the membranes were dried with paper tissue. An infinite dose of the drug (in practice approximately 1 ml) of the appropriate formulation was added to the donor compartment of each cell which was then occluded with a silicone-greased watch glass. 1 ml samples were taken, using an air displacement pipette, at 0 hours and at intervals over three days. An equivalent volume of replacement receptor solution was added to each cell following sampling. All samples were analysed by HPLC.

The mean flux (six cells) for the quick break foam formulation over the first 24 hours post-application was 43.3 $\pm$ 4.4 ug cm$^{-2}$ h$^{-1}$. The equivalent value for the gel formulation was only 15.1 $\pm$ 4.0 ug cm$^{-2}$ h$^{-1}$, i.e. less than one half of that achieved with the foam.

Example 5

This experiment measured the serum concentrations of BPAA after single topical application to the skin of rabbits from a 3% gel formulation and a 10% foam formulation.

The BPAA gel formulation employed has the same composition as that used in Example 3. The composition of the BPAA foam was as follows:

| Ingredient | % w/w in Foam |
|---|---|
| BPAA | 10.05 |
| Diisopropanolamine | 6.22 |
| Ethanol 96% | 43.20 |
| Purified Water | 26.95 |
| Polawax A31 | 3.56 |
| | 90.00 |
| Chlorofluorocarbon propellant | 10.00 |
| | 100.00 |

New Zealand White female rabbits (18) were randomly assigned to one of three groups each containing 6 animals. On the day before dosing, an area of the skin in the middle of the back slightly larger than 5 cm x 10 cm was shaved with clippers. Immediately prior to dosing, the animals were weighed and a blood sample removed from an ear vein into a plain tube. The dose of BPAA administered was 30 mg approximately accurately weighed irrespective of the formulation used. Both the gel and foam were applied with a spatula and were gently rubbed in. After application of each dose, the site of application was covered with a piece of aluminium foil which was held in place by elastic adhesive bandage. Blood samples were collected at 0.5, 1, 2, 3, 4, 6, 8, 12, 16, 24 and 48 hours after application of the doses.

On collection, the contents of blood tubes were mixed and allowed to stand at room temperature for 15 minutes, at 0-4°C for a further 15 minutes and then centrifuged at 1800 rpm for 15 minutes at 0-4°C. Serum was removed by pasteur pipette and frozen at -20°C.

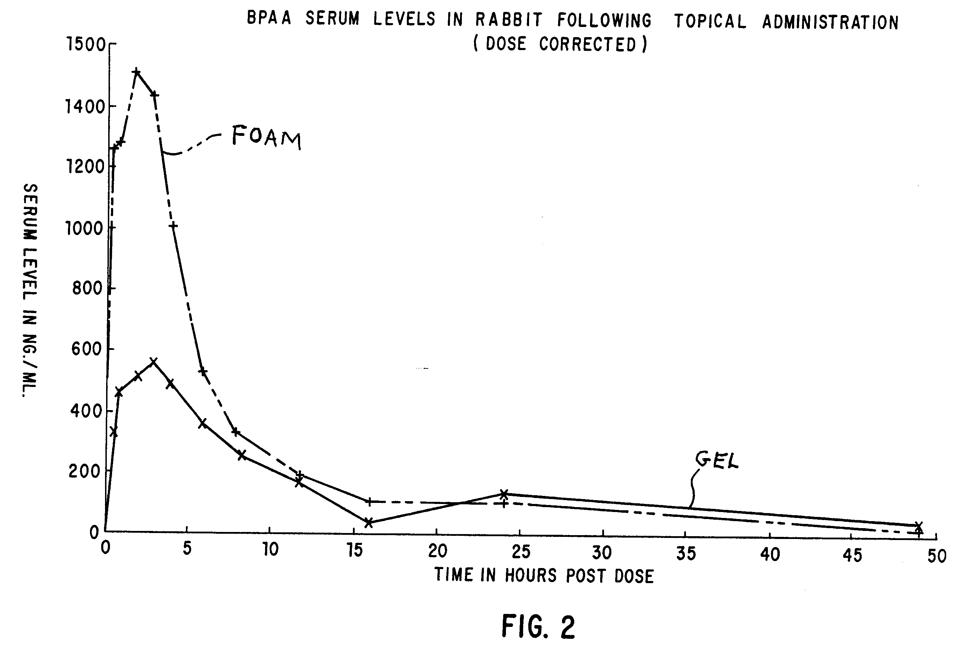

The results are shown graphically in Fig. 2, and are dose corrected.

From Fig. 2 it will be noted that the BPAA serum level was initially dramatically greater with the foam, being also three times greater at about 2-3 hours post administration.

However, after about 8 hours there were no significant differences in the BPAA serum levels between the two formulations.

**Claims**

**Claims for the following Contracting States : AT, BE, CH, LI, DE, DK, FR, GR, IT, LU, NL, SE, IE**

1. A pharmaceutical composition for topical application as an aerosol foam, comprising

   (a) 4-biphenylacetic acid (BPAA), or a pharmaceutically acceptable salt or ester thereof, in an amount of from 0.1 to 20% w/w calculated as the free acid, and (b) a carrier which comprises water, ethanol, an aerosol propellent and an ethoxylated stearyl alcohol surfactant, the relative proportions of said components of said carrier being such that the carrier is adapted to form a quick-breaking foam; the composition being maintained under pressure.

2. A composition according to Claim 1, comprising from 1.0 to 4.0% w/w of BPAA calculated as the free acid.

3. A composition according to Claim 1 or Claim 2, wherein the BPAA is present in the form of a pharmaceutically acceptable salt.

4. A composition according to Claim 3, comprising BPAA-ethanolamine salt.

# EP 0 535 327 B1

5. An aerosol dispenser containing a pharmaceutical composition according to any preceding claim.

**Claims for the following Contracting States : ES, PT**

1. A process for the preparation of a pharmaceutical composition for topical application as an aerosol foam, which comprises charging an aerosol dispenser with 4-biphenylacetic acid (BPAA), or a pharmceutically acceptable salt or ester thereof, and a carrier which comprises water, ethanol, an aerosol propellant and an ethoxylated stearyl alcohol surfactant in relative proportions such that the carrier is adapted to form a quick-breaking foam, said BPAA being charged in an amount of from 0.1 to 20% w/w calculated as the free acid, and said aerosol dispenser being closed to maintain said composition under pressure.

2. A process according to Claim 1, wherein said BPAA is charged in an amount of from 1.0 to 4.0% w/w, calculated as the free acid.

3. A process according to Claim 1 or Claim 2, wherein said BPAA is used in the form of a pharmaceutically acceptable salt.

4. A process according to Claim 3, wherein BPAA-ethanolamine salt is used.

5. A process according to any preceding claim, comprising the steps of:

   (a) preparing an alcoholic concentrate by dissolving said surfactant, BPAA and ethanolamine in ethyl alcohol,
   (b) separately charging said alcoholic concentrate and purified water into an open aerosol dispenser,
   (c) applying an actuator-valve assembly to said aerosol dispenser to close it, and
   (d) adding said propellant through the actuator valve.

**Patentansprüche**

**Patentansprüche für folgende Vertragsstaaten : AT, BE, CH, LI, DE, DK, FR, GR, IT, LU, NL, SE, IE**

1. Pharmazeutische Zusammensetzung zur topischen Anwendung in Form eines Aerosolschaums, umfassend:

   (a) 4-Bisphenylessigsäure (BPAA) oder ein pharmazeutisch akzeptables Salz oder einen pharmazeutisch akzeptablen Ester davon in einer Menge von 0,1 bis 20 % (G/G), berechnet als freie Säure, und

   (b) einen Träger, der Wasser, Ethanol, ein Aerosoltreibmittel und ethoxylierten Stearylalkohol als oberflächenaktives Mittel umfaßt, wobei die relativen Mengen der Bestandteile des Trägers so gewählt sind, daß der Träger in der Lage ist, einen schnell zusammenfallenden Schaum zu bilden;

   wobei die Zusammensetzung unter Druck steht.

2. Zusammensetzung nach Anspruch 1, umfassend 1,0 bis 4,0 % (G/G) BPAA, berechnet als freie Säure.

3. Zusammensetzung nach Anspruch 1 oder 2, worin BPAA in Form eines pharmazeutisch akzeptablen Salzes vorliegt.

4. Zusammensetzung nach Anspruch 3, umfassend das BPAA-Ethanolaminsalz.

5. Aerosolspender, enthaltend eine pharmazeutische Zusammensetzung nach einem der vorhergehenden Ansprüche.

**Patentansprüche für folgende Vertragsstaaten : ES, PT**

1. Verfahren zur Herstellung einer pharmazeutischen Zusammensetzung zur topischen Anwendung in Form eines Aerosolschaums, umfassend das Befüllen eines Aerosolspenders mit 4-Bisphenylessigsäure (BPAA) oder einem pharmazeutisch akzeptablen Salz oder Ester davon und einem Träger, der Wasser, Ethanol, ein Aerosoltreibmittel und ethoxylierten Stearylalkohol als oberflächenaktives Mittel umfaßt, in relativen Mengen, die es dem Träger ermöglichen, einen schnell zusammenfallenden Schaum zu bilden, wobei BPAA in einer Menge von 0,1 bis 20 % (G/G), berechnet als freie Säure, eingefüllt wird und wobei der Aerosolspender verschlossen wird, um die Zusammensetzung unter Druck zu halten.

2. Verfahren nach Anspruch 1, wobei BPAA in einer Menge von 1,0 bis 4,0 % (G/G) BPAA, berechnet als freie Säure, eingefüllt wird.

3. Verfahren nach Anspruch 1 oder 2, wobei BPAA in Form eines pharmazeutisch akzeptablen Salzes verwendet wird.

4. Verfahren nach Anspruch 3, wobei das BPAA-Ethanolaminsalz verwendet wird.

5. Verfahren nach einem der vorhergehenden Ansprüche, wobei man:

(a) ein alkoholisches Konzentrat herstellt, indem man das oberflächenaktive Mittel, BPAA und Ethanolamin in Ethylalkohol löst,

(b) das alkoholische Konzentrat und gereinigtes Wasser einzeln in einen offenen Aerosolspender einfüllt,

(c) den Aerosolspender mit einem Betätigungsventil versieht, um diesen zu verschließen, und

(d) das Treibmittel durch das Betätigungsventil zugibt.

**Revendications**

**Revendications pour les Etats contractants suivants : AT, BE, CH, LI, DE, DK, FR, GR, IT, LU, NL, SE, IE**

1. Composition pharmaceutique à appliquer localement sous forme d'une mousse en aérosol, comprenant (a) de l'acide 4-biphénylacétique (BPAA) ou un sel ou ester pharmaceutiquement acceptable de cet acide en une quantité de 0,1 à 20 % en poids/poids calculée sur la base de l'acide libre, et (b) un véhicule qui comprend de l'eau, de l'éthanol, un propulseur pour aérosol et, comme agent tensio-actif, un alcool stéarylique éthoxylé, les proportions relatives de ces composants du véhicule étant telles que le véhicule soit rendu apte à former une mousse se cassant rapidement, la composition étant maintenue sous pression.

2. Composition suivant la revendication 1, comprenant 1,0 à 4,0 % en poids/poids de BPAA, exprimé sur la base de l'acide libre.

3. Composition suivant la revendication 1 ou la revendication 2, dans laquelle le BPAA est présent sous la forme d'un sel pharmaceutiquement acceptable.

4. Composition suivant la revendication 3, dans laquelle on utilise le sel d'éthanolamine du BPAA.

5. Distributeur d'aérosol contenant une composition pharmaceutique suivant l'une quelconque des revendications précédentes.

**Revendications pour les Etats contractants suivants : ES, PT**

1. Procédé de préparation d'une composition pharmaceutique à appliquer localement sous forme d'une mousse en aérosol, qui comprend les étapes consistant à charger, dans un distributeur d'aérosol, de l'acide 4-biphénylacétique (BPAA) ou un sel ou ester pharmaceutiquement acceptable de cet acide et un véhicule qui comprend de l'eau, de l'éthanol, un propulseur pour aérosol et, comme agent tensio-actif, un alcool stéarylique éthoxylé, dans des proportions relatives choisies de manière que le véhicule soit rendu apte à former une mousse se Cassant rapidement, le BPAA étant chargé en une quantité de 0,1 à 20 % en poids/poids calculée sur la base de l'acide libre, et le distributeur d'aérosol en question étant fermé de manière à maintenir la composition sous pression.

2. Procédé suivant la revendication 1, dans lequel le BPAA est chargé en une quantité de 1,0 à 4,0 % en poids/poids, exprimée sur la base de l'acide libre.

3. Procédé suivant la revendication 1 ou la revendication 2, dans lequel le BPAA est utilisé sous la forme d'un sel pharmaceutiquement acceptable.

4. Procédé suivant la revendication 3, dans lequel on utilise le sel d'éthanolamine du BPAA.

5. Procédé suivant l'une quelconque des revendications précédentes, qui comprend les étapes consistant :

    (a) à préparer un concentré alcoolique par dissolution de l'agent tensio-actif, du BPAA et d'éthanolamine dans de l'alcool éthylique,
    (b) à charger séparément ce concentré alcoolique et de l'eau purifiée dans un distributeur d'aérosol ouvert,
    (c) à adapter un ensemble à valve d'actionnement sur le distributeur d'aérosol pour le fermer et
    (d) à introduire le propulseur par la valve d'actionnement.

## FIG.1.

### DIFFUSION OF FELBINAC ACROSS HAIRLESS MOUSE SKIN

3% GEL. ○ — — — —     3% FOAM $ ——— -

% DOSE CROSSING SKIN

TIME IN DAYS

EP 0 535 327 B1

BPAA SERUM LEVELS IN RABBIT FOLLOWING TOPICAL ADMINISTRATION
(DOSE CORRECTED)

FIG. 2